(19) **Europäisches Patentamt**
**European Patent Office**
**Office européen des brevets**

(11) **EP 4 681 650 A1**

(12) **EUROPEAN PATENT APPLICATION**

(43) Date of publication:
**21.01.2026 Bulletin 2026/04**

(21) Application number: **24189562.2**

(22) Date of filing: **18.07.2024**

(51) International Patent Classification (IPC):
**A61B 6/46** (2024.01)          **A61B 6/00** (2024.01)

(52) Cooperative Patent Classification (CPC):
**A61B 6/542; A61B 6/469; A61B 6/54; A61B 6/545**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventor: **SAALBACH, Axel**
**Eindhoven (NL)**

(74) Representative: **Philips Intellectual Property & Standards**
**High Tech Campus 52**
**5656 AG Eindhoven (NL)**

(54) **AUTOMATIC X-RAY EXPOSURE DEVICE SELECTION**

(57)     System (FS) and related method of facilitating operation of a medical X-ray imaging apparatus having plural exposure control devices (SN, ECD1-5). The system comprises an input interface (IN) through which is receivable input data (*m*) pertaining to an acquisition operation to be performed in relation to a region of interest (ROI). A selector module (SM) is capable to provide a selection (*s*), based on the input data, of one or more of the plural exposure control devices (SN, ECD) for activation during the acquisition operation. The selector module (SM) is based on a trained machine learning model (M).

FIG. 4

EP 4 681 650 A1

**Description**

FIELD OF THE INVENTION

The invention relates to a system of facilitating operation of a medical X-ray imaging apparatus, to a related method, to an imaging arrangement, to a machine learning training system, to a training data procurer system, the related machine learning methods, to a computer program element and to a computer readable medium.

BACKGROUND OF THE INVENTION

Medical imaging refers to a number of different imaging techniques that allow obtaining imagery of structural or functional details of within a patient's body. It is in general, a non-invasive technique and can be deployed rapidly. Medical imaging is among the most useful tool in the medic's arsenal as it informs usefully therapy and/or diagnosis. It can also be used for training purposes of medical personnel.

A celebrated and much used technique in medical imaging is X-ray based imaging. In this imaging technique, developed by Conrad Roentgen in the late 19th century, a region of interest of the patient to be imaged is exposed to X-radiation. This X-radiation interacts with the tissue inside the patient. The X-radiation upon such exposure emerges distal side of the patient, and is then registered in an X-ray sensitive detector as a distribution of intensity values, which can be used to image the patient's internal anatomy. This is because the measured registered intensity vary spatially is with a type of material or its amount that was encountered by the X-radiation on its paths through patient. The measurements so obtained may be converted into digital imagery which can then be rendered for view on the display device. Medical personnel such as a radiologist or other may then examine the imagery to so inform diagnosis and/or therapy or whatever the task at hand.

Useful a tool as X-rays may be for imaging, this still comes at a cost. The mentioned exposure to radiation deposits energy in the human tissue which can lead to undesirable side effects such as injury, burns, but also cancer if exposure exceeds certain known dosage limits. The energy deposited is in general a function of length of exposure and the energy of the X-ray source used. Guidelines have been developed based on medical knowledge to strike a balance between the downsides of radiation to health, and its usefulness in producing imagery of sufficient contrast. The proper use of radiation and its energy where it needs to be directed to, length of exposure etc, is as said put down in guidelines, but it is also to some extent part of the medic's knowledge.

Safety measures have been into place, ranging from lead aprons, collimators, etc, to measurement devices, also referred as Automatic exposure control (AECs). Such AEC devices may be arranged at the imager or in the exam room to measure radiation, and, if a safety level of X-ray dose is exceeded, any ongoing X-ray exposure is halted, or a requested such X-ray exposure is declined from going forward, etc.

Despite such dosage safety measures in place and used by some manufacturers, it has been observed at times that radiation dosages are still exceeded in practice which can have the said adverse health effects on the patient or on medical personnel, such as in interventional X-ray imaging, image-guided therapy applications (IGT), C-arm imaging, fluoroscopy, etc.

SUMMARY OF THE INVENTION

There may therefore be a need for improved, safer operation, in particular of an X-ray based imaging modalities. The below described is with particular use for radiography that is planar X-ray imaging but uses and benefits are not so confined and, indeed, application in the tomographic realm such as for computer tomography, scanners or interventional imaging equipment such as C or U-arms are also envisaged herein. However, for the sake of definiteness the following examples will mainly refer to radiography X-ray imaging, which is purely projection imaging, different from tomographic imagery, where multi-directional projection imagery is computationally combined into sectional imagery.

An object of the present invention is achieved by the subject matter of the independent claims where further embodiments are incorporated in the dependent claims. It should be noted that the following described aspect of the invention equally applies to the related method, to imaging arrangement, to training system, the training data procurer system, the related methods, to the computer program element and to the computer readable medium.

According to a first aspect of the invention there is provided a system of facilitating operation of a medical X-ray imaging apparatus having plural exposure control devices, comprising:

an input interface through which is receivable input data pertaining to an acquisition operation to be performed in relation to a region of interest;
a selector module capable to provide a selection, based on the input data, of one or more of the plural exposure control devices for activation during the acquisition operation, wherein the selector module is based on a trained machine learning model.

In embodiments, the system is capable of providing output data through an output interface, the said output data indicative to the said selection.

In embodiments, the output data includes a graphics display capable of visualizing, on a display device, the said output data.

In embodiments, the system includes a user interface capable of allowing user to confirm the said selection.

In embodiments, the selector module is further based on a rule-based decision logic.

The system of any one of the preceding claims, wherein the input data includes non-ionizing radiation-based imagery acquirable by a scout camera of at least a part of the region of interest.

The scout camera may be any one of more of RGB, RGB-D camera, but also LiDAR, and others still.

In embodiments, the region of interest includes at least a part of an anatomy of a patient to be imaged.

In another aspect there is provided an imaging arrangement, comprising the system of any one of the preceding claims, and further comprising: i) the imaging apparatus, ii) the user interface; iii) the display device, iv) the scout camera, v) at least one of the plural exposure control devices.

In another aspect there is provided a method for facilitating operation of a medical X-ray imaging apparatus having plural exposure control devices, comprising:

receiving input data pertaining to an acquisition operation to be performed in relation to a region of interest; and
selecting a selection, based on the input data, of one or more of the plural exposure control devices for activation during the acquisition operation, wherein the selecting is based on a trained machine learning model.

In yet another aspect there is provided a training method for training, based on training data, the machine learning model.

In another aspect there is provided a method of providing the training data for use in the training method.

In another aspect there is provided a computer program element, which, when being executed by at least one processing unit, is adapted to cause the processing unit to perform any one of the methods.

In another aspect there is provided at least one computer readable medium having stored thereon the program element, or having stored thereon the trained machine learning model.

Thus, what is proposed herein is a system that facilitates a safer operation of an X-ray imagery where a risk of over-exposure to radiation is reduced or avoided altogether. Such facilitation is provided herein by computing-assisted, guided, selection of dose exposure device(s), also referred to as AECs herein.

The trained machine learning ("ML") model can be configured for end-to-end, where input is classified into the selection data directly by the model itself. However, a staged setup such as with a rules-based engine as backend are not excluded herein, where model computes intermediate data from input data, and this is then processed/converted or otherwise into the selection data as by the rules-based backend-end engine. For example, the ML model may be configured to segment input scout imagery provided by scout camera into a scene, and this is then processed in a rules-based manner to obtain the AEC sensor selection.

In another aspect there is provided a computer program element, which, when being executed by at least one processing unit, is adapted to cause the processing unit to perform the method as per any one of the above mentioned embodiments.

In another aspect still, there is provided a computer readable medium having stored thereon the program element.

"*user*" relates to a person, such as medical personnel or other, operating the imaging apparatus or overseeing the imaging procedure. In other words, the user is in general not the patient, although in autonomous setting patient may be asked to perform or participate in some functions that previously were carried out by user.

"*object*" is used herein in the general sense to include animate "objects" such as a human or animal patient, or anatomic parts thereof but also includes inanimate objects such as an item of baggage in security checks or a product in non-destructive testing. However, the proposed system will be discussed herein with main reference to the medical field, so we will be referring to the "object" as "the patient" or a part of the patient, such as an anatomy or organ, or group of anatomies or organs of the patient.

In general, the "*machine learning*" includes a computerized arrangement that implements a machine learning ("ML") algorithm to adapt a machine learning model that is configured to perform a task. This adaption is called "training. Task performance by the ML model improves measurably, the more (new) suitably varied training data is used to train the model. The performance may be measured by objective tests when feeding the system with test data. The performance may be defined in terms of a certain error rate to be achieved for the given test data. See for example, T. M. Mitchell, "Machine Learning", page 2, section 1.1, McGraw-Hill, 1997.

BRIEF DESCRIPTION OF THE DRAWINGS

Exemplary embodiments of the invention will now be described with reference to the following drawings, which, unless stated otherwise, are not to scale, wherein:

Fig. 1 shows a schematic diagram that illustrates an X-ray imaging arrangement including an X-ray imaging apparatus as may be used herein in embodiments;
Fig. 2 shows a schematic block diagram of an automated exposure control device as may be used in embodiments envisaged herein;
Fig. 3 illustrates operation of an X-ray imaging apparatus in connection with related exposure control devices;
Fig. 4 shows a block diagram of a system facilitating safe operation of an X-ray imaging apparatus;
Fig. 5 shows a flow chart of a computing implemented method of facilitating safe operation of an X-ray imaging apparatus;
Fig. 6 shows a flow chart of a method of operating an imaging apparatus;
Fig. 7 shows a block diagram of a machine learning model and its architecture which may be used in embodiments described herein;
Fig. 8 shows a block diagram of components of a training system for training a machine learning model;
Fig. 9 shows a computing implemented method of obtaining training data for the training of a machine learning model; and
Fig. 10 shows a flow chart of a computing implemented method of training a machine learning model.

DETAILED DESCRIPTION OF EMBODIMENTS

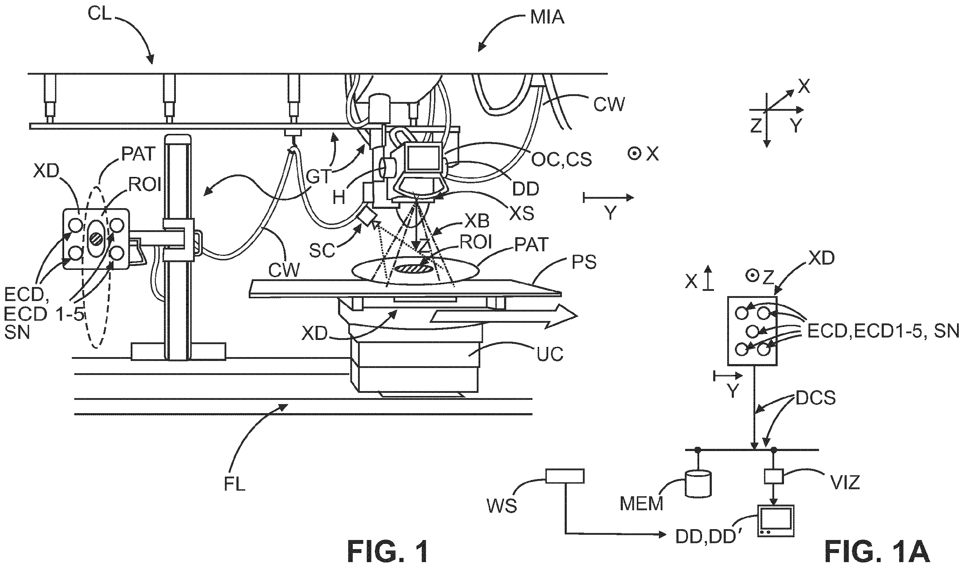

Reference is first made to Fig. 1 which illustrates various components of a medical imaging arrangement MIA as may be used herein. Broadly, the medical imaging arrangement MIA includes a medical imaging apparatus ("imager") IA in an exam room. The imager IA is operable to acquire imagery at its X-ray detector XD, in particular, projection imagery, of a region of interest ROI of a patient PAT. Operation of the imaging apparatus may be controlled by a user from an operator consol OC.

The imagery so acquired may be forwarded from the imager IA, in particular from the imager IA's detector XD, through a data communication system DCS to any data consumer or for storage. The data communication system DCS may be wired, wireless or a hybrid of both. Image data acquired during an imaging session by the imaging apparatus IA may be otherwise processed by a computing system.

A computing system CS may be one such data consumer and may be operable to process the imagery so acquired, such as for view on a display device DD, DD' as caused by a visualizer VIZ, for storage in a volatile or non-volatile memory MEM, or for processing by any other data consumer such as by an image analyzer to aid user in diagnosis, or on any other way. The said non-volatile memory may include a medical image database such as a PACS or other, that may be maintained by a medical facility, a GP practice, a hospital or clinic. The computing system CS may be part of the operating console OC.

The display device DD may be integrated into the operator consol OC of the imager IA for immediate on-site viewing as shown in the upper portion of the Figure, but this may not be so in all embodiments, but the display device may be located elsewhere in the exam room, as needed, or may be part of a mobile computing device. Instead, or as an alternative, the visualization may be effected off-site on a remote display device DD', eg of a workstation WS or mobile device, possibly situated in a different room than the exam room. A radiologist may review such imagery on such a workstation WS or any other computing device, in a "reading session" to prepare reports that may inform therapy.

Broadly, as will be explored in more detail below, the imaging arrangement MIA may include one or more exposure control devices ECD, ECD 1-5 that monitor dose exposure of patient, and may effect precluding or interrupting such exposure, if a certain impermissible dosage level is reached. One such arrangement is shown in the insert Fig. 1A, where such devices ECD1-5 are arranged at, in, or behind, the detector XD. The imaging arrangement MIA may include a novel facilitator system FS that facilitates safe operation of such imaging arrangement equipped with such exposure control devices ECD, ECD 1-5. The facilitator system FS may be computing based. It may be fully or partly implemented by the computing system CS of the imaging arrangement MIA, by the operator console OC, or may be otherwise implemented or may be part of any other suitable computing system or device, as needed.

Before exploring operation of the facilitator system FS in more detail, operational aspects of the imaging arrangement MIA are presented first to so assist the later more in-depth exploration of the said facilitator system FS.

The particular imaging arrangement as shown in Fig. 1 is for illustration only. For example, the Figure illustrates a radiography imaging system that is operable to acquire projection imagery with a fixed detector (left) and a portable detector (right). In fixed detector setting there is usually line of sight (the detector is visible), whilst in portable detector settings, the detector may not be visible, eg detector may be slid under the patient support top, or in mobile imaging where patient is indeed lying on the detector. In mobile- detector settings, patient PAT may lie, sit or otherwise on a patient support PS which is arranged on an under-carriage UC. In such settings , it is not necessary for the whole of the patient to be resting on the support: it may be sufficient for the region of interest ("ROI") to be resting thereon, such as in orthopedic imaging where patient is asked to position their hand, arm, elbow, foot, leg on the patient support. However, the patient PAT/ROI may not necessarily so lie or reside on such a patient support PS, but patient PAT may instead stand during imaging as may be done with the fixed detector setting shown to the left of Fig. 1. For example, the patient is asked to stand in a predetermined area of the exam room, such as in chest X-ray, whilst the X-ray imagery is acquired, with the X-ray detector mounted in a stand or gantry.

The arrangement MIA as illustrated in Fig. 1 may be configured for dual use, that is for fixed detector and portable detector-imaging, but this may not be so necessarily. In simpler embodiments, the arrangement MIA is one but not the other. For example, it may be arranged for either supported or for orthostatic imaging, as needed. In the dual use setup as illustrated in Fig. 1, the same (single) detector XD, in this case a portable unit, may be used in either fixed detector or portable detector imaging. Similarly, it may be the same X-ray source XS, that may be used in fixed detector and portable detector-imaging . Alternatively, in systems for such dual use, there is a dedicated detector XD and/ source XS of either use.. More than one detector XD may be used, such as in bi-plane imagers, also envisaged herein.

An imaging co-ordinate system may be defined $(X,Y,Z)$ , where axis Z follows roughly the center beam of the primary X-ray beam X as it impinges on the center point of the X-radiation sensitive surface of the detector. Said surface may planar or curved. The other spatial directions $X, Y,$ of which one (axis X) extends to the drawing plane of Fig. 1, are generally the two spatial dimensions of the projection imagery that is obtainable.

Instead of the room-based imaging arrangement as in Fig. 1, mobile X-ray imagers IA are also envisaged herein, and so are tomographic X-ray modalities, such as the mentioned C-arm or diagnostic CT scanners, instead of the purely projection-based setup, illustrated in Fig. 1.

Cablework CW may supply power and/or control signaling to the various components, in particular an X-ray source XS of the imager IA, the detector XS, operator console OC, or to the computing system CS or facilitator system FS. Instead of such common cablework CW for power supply, the detector XD or other of said components or others still, may have its own on-board power source such as (but not only) in portable detector embodiments.

Broadly, and turning now in more details to aspects of the imaging session/ image acquisition, the imager IA's X-ray source XS is operable, upon energizing, to issue forth an X-ray beam XB at a pre-set energy. Specifically, during imaging, X-ray beam XB emanates from a focal spot (not shown) of the X-ray source XS, passes through the patient PAT, in particular through the region of interest, and is then incident on the X-ray detector XD. The X-ray detector XD is arranged during imaging across and opposite the X-ray source XS, such that the region of interest resides during imaging in a 3D space in between the X-ray source XS and the X-ray detector XD.

The X-ray source XS and/or the X-ray detector XD may be arranged in a respective gantry GT, such as is shown in Fig. 1.

In some embodiments such as in tomographic C-arm imagers, which is not shown in Fig. 1, the X-ray source and the X-ray detector are arranged on a common rigid gantry together which by rotation of the gantry allows acquiring projection imagery from multiple directions. However, a simpler radiography system as shown in Fig. 1 does not need such common gantry where both X-ray source and X-ray detector are connected. Indeed, there is no physical connection in some embodiments as shown in Fig. 1 between the X-ray detector XD and the X-ray source or in mobile X-ray imagers.

The X-ray source may be arranged in a separate housing H on the gantry GT, whilst the X-ray detector XD may be arranged as an autonomous standalone unit in said portable setup, preferably with communication interfacing (wired and/or wireless) for forwarding through communication system DCS to data consumers. It may be portable or fixed as mentioned earlier. For example, it may be fixed to a gantry arranged as a wall or floor-stand or may be integrated in the patient support PS, removable or fixedly, or in any other way. In the portable embodiment as shown in Fig. 1, the detector may be slid under the patient into an opening in the under-carriage to come to rest on the patient support. The patient support is preferably sufficiently transparent to X-radiation, so that the image quality is not marred by this arrangement. A bucky-tray may be used, integrated under the tabletop to receive the detector XD for use. Any other arrangement may be used to so receive the detector. Thus, in such embodiments, the table PS's undercarriage UC may serve as the detector XD's gantry GT for carrying the detector XD.

The X-ray detector XD may include plural X-ray-sensitive spatially fixed X-ray sensitive detector pixels, arranged in a matrix layout such as is envisaged in embodiment for 2D detectors, such digital detectors of flat panel design. The X-ray detector XD may be of the direct conversion type, or indirect conversion type. Both may use semiconductors as transducers. The latter further uses a scintillator, whilst the earlier does not. The detector pixels detect X-ray radiation as intensities, after the X-ray radiation's passage through patient tissue.

The mentioned intensities that are registered during imaging vary with tissue type or amount of tissue in the radiation's path. The radiation is transduced by the X-ray pixels into said intensities as respective, spatially resolved, pixel values. They may read out via read-out circuitry, and may be A/D converted into spatial resolved data. Such data may be supplied by detector XD as a 2D matrix set of numbers which represents the image data. This spatial data can then be passed on by the data communication system DCS for processing, storing or may be otherwise used, as the case may be. Figure illustrates radiation XB as primary radiation. However, there is also in general secondary radiation, which is caused by scattering effects caused by patient tissue, or by radiation interacting with other material or object in the exam room. Thus, X-radiation may be detectable not only at the detector, but at other places in the exam room.

Whilst the above signal processing chain for image generation was explained at the example of native digital imaging, which is mainly envisaged herein, the present disclosure is not so confined. Instead, more traditional, "pre-digital" imaging with the detector XD arranged as sealed cassette including X-ray sensitive film where the actual X-ray acquisition occurs, are also envisaged herein in alternative embodiments.

Imaging geometry may be adjustable. Imaging geometry relates to the mutual spatial 3D arrangement or configuration between i) the region of interest to be imaged, ii) the X-ray source XS and iii) the X-ray detector XD. The imaging geometry may be adjusted to ensure that the ROI is (wholly) in the field of view of the X-ray imager IA. Thus, the imaging geometry ought to be adjusted prior X-ray exposure so that the ROI is within the beam XB. The imaging geometry may be adjusted by adjusting any one or more of roll, pitch and yaw of the X-ray source and/or of the detector's radiation sensitive surface, respectively. Translation along any one or more of the spatial axes $X, Y, Z$ may also be effected, such as in a tracked arrangement or other. This can be done electronically by actuators. Such actuators may be arranged as one or more servo-motor. Such servo-motors may effect motion along respective axes and/or rotations thereabout, so that the position and/or orientation of the X-ray detector and/or the X-ray source may be adjusted automatically. Alternatively, such imaging geometry adjustment may be assisted through such servo motors, as the user operates a suitable user interface at operator consoles, such as a joystick for example, to issue related control signals. However, a more "hands-on" arrangement is not excluded where handles, as illustrated in Fig. 1, are arranged at the detector and/or at the housing of the X-ray source which allows a user to change the imaging geometry so that irradiation of the region of interest can be assured. As shown in Fig. 1, imaging geometry adjustment may be facilitated by having detector XD and/or source XS arranged on tracks or on articulated arms, etc, either floor FL-, ceiling CL or wall mounted, as needed. Further or alternative imaging geometry adjustments may be done by operating a collimator (if any) or by adjusting the ROI-to- source XS or -ROI-to- detector XD distance, for example by adjusting height of patient support PS.

The patient PAT, in particular the region of interest, may also be suitably positioned in the examination region by instruction or guidance/promoting through the medical user, or in an automated fashion, through a suitable patient-assistance system that may be video-based, sound-based, or any other.

As will be explored in more detail, the imaging arrangement may include in some (but not all embodiments) a scout camera SC. This camera may be configured for non-ionizing imaging, as opposed to X-ray imaging which is ionizing. The one or more scout camera CS may include any one or more of an optical camera, infra-red camera, depth-sensing camera. The scout camera may be arranged suitably either on the gantry, at the housing of the X-ray source, or anywhere in the exam room, ceiling or wall mounted. The field of view of camera SC can be adjusted to capture non-ionizing imagery of the patient, or the ROI, as residing in the examination region, such as standing, sitting or lying on the patient support.

As mentioned earlier, useful as X-radiation is for imaging, it comes at a dosage cost which can be a health hazard. It is therefore an objective to restrict exposure to X-radiation as much as possible and to still ensure sufficient contrast in the obtained imagery. To this end, the above-mentioned set of plural exposure control devices ECD, ECD1-5 may be used in the medical arrangement MIA, for example in a manner as illustrated in inset Fig. 1A.

The exposure device ECD may be arranged as automated exposure control devices ("AEC"), and for such they may be referred herein. Plural such AEC devices spatially distributed in the medical arrangement MIA may be used. They are suitably placed to be able to detect primary and/or secondary radiation for the majority, if not all, envisaged imaging protocols. Five such devices ECD 1-5 are illustrated, but this is exemplary as any other number of such two or more devices ECD 1-5 are envisaged herein. In some cases, for very detailed dose monitoring for a large number of imaging protocols, the number of such devices ECD may be in the double digits, or much higher still if some of the detector XD's pixels are to be used themselves as AEC devices, an option also envisaged herein in some embodiments. In extreme cases, a single detector pixel, or a relatively small group of such pixels, neighboring or not, may be designed for use a AECs in some envisaged embodiments.

Each of the plural exposure control devices ECD may be considered a separate autonomous device that can co-operate with the imaging apparatus, in particular with the X-ray source thereof through suitable communication interfaces. The exposure control devices ECD may include spatially discretely arranged sensors SNj ($j>1$, eg $j=5$ as illustrated in the Figure) that are suitably arranged at a respective, in general fixed, position throughout the medical imaging arrangement as mentioned, in a manner to be able to pick up radiation for intended imaging protocols. They may be arranged on the floor, or may be ceiling mounted, wall mounted, mounted on the gantry or on any other object. Preferably however, the exposure control devices ECDs are either placed in front or behind the detector or are part the detector itself acts as such device ECD(s). Some devices SN1-5, ECD1-5, ECD may be mounted on, or in, the housing (not shown) of the X-ray detector, as illustrated in Fig. 1, as needed, and depending on the imaging use scenario intended to monitor dose for. As its name implies, each of the exposure control devices is operable to control, and thus restrict, the amount of X-ray radiation to which the patient is exposed to in imaging. In general, the devices ECD include one or more X-ray sensors and these are spatially so distributed that at least one such sensor is capable of detecting radiation to which patient would be exposed to, for any one or more (in particular all) imaging protocols intended to be run at the particular imaging arrangement MIA or imager IA.

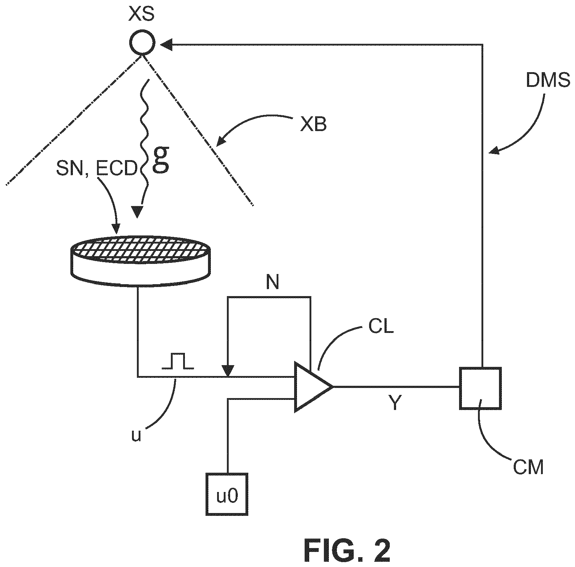

Operation of a dose monitoring system DMS comprising such (one or more) exposure control devices ECD is now illustrated in the block diagram of Fig. 2, to which reference is now made. Only one such exposure control devices is illustrated therein with the understanding that the other ones of the plural ECDs are similarly arranged. Thus, the following applies to each of such exposure control devices used in imaging arrangement MIA. In the following, and throughout this disclosure, a reference herein to an exposure control devices ECD of the plural such devices is in particular a reference to one or more sensors, and it is such X-ray sensors that are spatially arranged and distributed in, at or throughout the imaging arrangement IA. Thus, any reference herein to a particular exposure control device is in particular to be construed as reference to its one or more associated X-ray sensors. In more detail, each of the separate exposure control devices ECD may be arranged as a an X-ray sensitive sensor(s) SN. In the following reference, may be made to such sensor SN instead of the exposure control devices ECD. Thus, "sensor SN" and exposure control devices ECD, and AEC, shall be used interchangeably herein.

Such sensors SN are operable to detect X-ray radiation, in particular gamma photons $\gamma$, of which the X-ray beam XB is made up. The sensor SN converts the incoming radiation depending on its energy into an electrical signal $u$. Each or some such sensors SN may be made up as an ionization-chamber. Such chambers or receptacles include an amount of suitable gas. The chamber is arranged between a pair of electrodes, and an operating voltage is applied across the chamber and hence the gas in it. Incoming X-ray energy causes ions to be formed in the gas, which diffuse, driven by the operating voltage applied, to the electrode, thus causing an electrical signal, such as voltage or amperage which can be picked up by a voltmeter or amperemeter. The electrical signal varies in magnitude with the energy of the X-radiation may. The measured electrical signal may be integrated over time as indeed is envisaged herein to obtain a measure for the accumulated energy over time so deposited in patient tissue by the X-radiation.

The so detected accumulated or time integrated measurement signal $u$, is compared by control logic CL against a safety threshold value $U0$ held in a memory (not shown. This safety threshold value $U0$ may be pre-set, or may be user adjustable, as needed. The control unit CL compares the measured signal $u$ indicative of over time accumulated X-ray radiation detected by the sensor SN. If the threshold $U0$ is not violated, in particular is not exceeded, the exposure control device ECD allows the imaging to continue, in particular, allows exposure to X-radiation to continue, start or resume. If, however, the measured value $u$ violates the threshold $u0$ according to a pre-defined policy, such as exceeds the threshold $U0,$ a control module CM is instructed by logic CL through a suitable control signal. The logic CL may then interface with circuitry of the X-ray source XS to cause radiation to stop from issuing forth from the source XS . In particular, the X-ray source is switched off or otherwise disabled so that no more exposure can occur. Thus, the imaging may be interrupted by operation of monitoring system based on signalling from one or more of the ECD1-5 sensors SN1-5. As said earlier, a given sensor SN may also be referred to herein simply as "AEC" (sensor/device).

The monitoring system DMS may process as described above radiation doses as sensed at various discretely located sensors SN, suitably spatially distributed as various locations at, in, or around, the imaging arrangement MIA, as described above. The dose control behavior, as mediated by control module CM, may proceed accordingly to the said safety policy or other. For example, if dosages from plural sensors SNj are detected, it is the overall detected radiation doses (such as their sums) that is used to trigger abortion of the X-ray exposure. Alternatively, it is the dosage exceeding at a single sensor that is determinative of whether or not to so abort. Alternatively still, it is the so sensed dosages that are computationally combined into a score, such an average, or weighted average or other, that is so determinative.

Whilst both, the X-ray detector XD and the various AEC sensors $SN_j$ are X-ray sensitive, they may differ in their construction and purpose. The X-ray detector is used for image data acquisition as described, whilst the AEC sensors $SN_j$ are not. They are used instead for X-ray dosage monitoring and control. The AEC sensors $SN_j$ may be configured to react faster to radiation than the X-ray (imaging) detector XD. Operation of the X-ray (imaging) detector XD may be based on electron-hole-migration in latticed solids causable by impinging X-ray, in particular in crystalline semiconductors, whilst the faster sensors SN of AECs, may rely on ionization of gaseous matter. Indeed, fast reaction is preferred for the sensors SN. A portion(s) (detector pixels) of a sufficiently fast X-ray detector may be used instead as AEC sensor as described herein in . Thus, in some embodiments, parts of the functionality of the AEC sensors SN may be integrated in the X-ray detector itself. However, arrangement in functionally and spatially separated entities as described may be preferred, on account of more favorable reaction/switching times and cost, or other factors.

As mentioned, the sensors SN of the AECs are arranged fixedly in the exam room, with one or more possibly integrated into the medical imaging arrangement at fixed locations. So, in general, position and/or orientation of those sensors may not be changed unless the particular equipment into which they are integrated of course change spatially. For example, as shown, in Fig. 1A, five such AECs sensors SN, SN1-5 are integrated into the housing of the X-ray detector XD, or are placed on such housing. If detector XD serves as AEC, the region of interest may be adapted based on the patient PAT.

For meaningful diagnostic imaging, the patient needs to be positioned properly relative to the X-ray source and/or detector as mentioned. Thus, the imaging geometry must be sound as put down in protocols, imaging guidelines, etc. This can be done, as said, by changing orientation position of the X-ray source and/or detector, but also by positioning the patient suitably. It ought to be ensured that at least the region of interest ROI will come to be irradiated by the X-ray beam XB sufficiently to obtain diagnostic imagery. As the medical imaging arrangement is preferably arranged so that it can support a plural number of different medical imaging protocols, the exposure control devices ECDs are distributed accordingly, guided by medical knowledge to be able to perform their safeguarding/monitoring function as intended. In other words, the layout of the exposure control devices ECD's distribution is chosen so as to cater for multiple imaging uses, protocols, purposes, anatomy, patient physiology, etc.

Thus, in most cases, for any given imaging task, it is not necessary, and indeed not advised or useful, to use all of the ECD sensors SN. Only a sub-set of them may be used ("activated") for any given imaging task. The subsets may differ for different tasks. Thus, different combinations from the total of plural available AEC sensors $SN_j$ may be used for some imaging tasks/protocols. Each such combination may be referred to herein as an activation pattern. Activation of an AEC sensor $SN_j$ may refer to monitoring the exposure during imaging and comparing the electrical measurement u signal provided by the sensor against the safety threshold, and applying the radiation exposure policy by logic LC to enforce same when the violation occurs, such as by aborting, impeding, or halting the X-ray exposure by issuing a suitable switching signal, or by other suitable interference to this effect. For example, the power to the X-ray source may be switched off, or a breaker grid is switched on. Such grid may be arranged within the X-ray tube XS between tube's anode and cathode. It may be electrified to so prevent an electron beam generated inside the tube to each the X-ray tubes anode disk, thereby preventing X-ray radiation from egressing tube. In contrast, any such AEC sensor is said to be "deactivated" or "not active", if its electrical measurement u is ignored by the logic CL, and not taken into account. In addition, or instead, the respective AEC sensor may be placed in hibernation mode, so that it is unable to generate such measurement in the first place. Any such AEC sensor is either activated or de-activated. A specific activation pattern determined a counterpart deactivation pattern. Thus, we will be referring herein to a selection of activation patterns, instead of to the equivalent notion of selection of counterpart deactivation pattern. Thus, a selection of AEC sensors SN, as used herein, represents a corresponding activation pattern. In addition, or instead, to this binary, "hard", activation pattern as described above (AEC sensors SN either ignored or not), a more general, weighted, "soft" activation pattern may be envisaged. In such soft activation pattern, a weighting of sensor outputs may be contemplated. Thus, the proposed system may help user to (re-)weigh the various sensors SN for dose monitoring use. However, for the sake of definiteness and illustration, in this disclosure main reference is made to the hard activation pattern, but all that is described herein is of equal application for the soft activation pattern, and such soft activation pattern as indeed envisaged herein in embodiments.

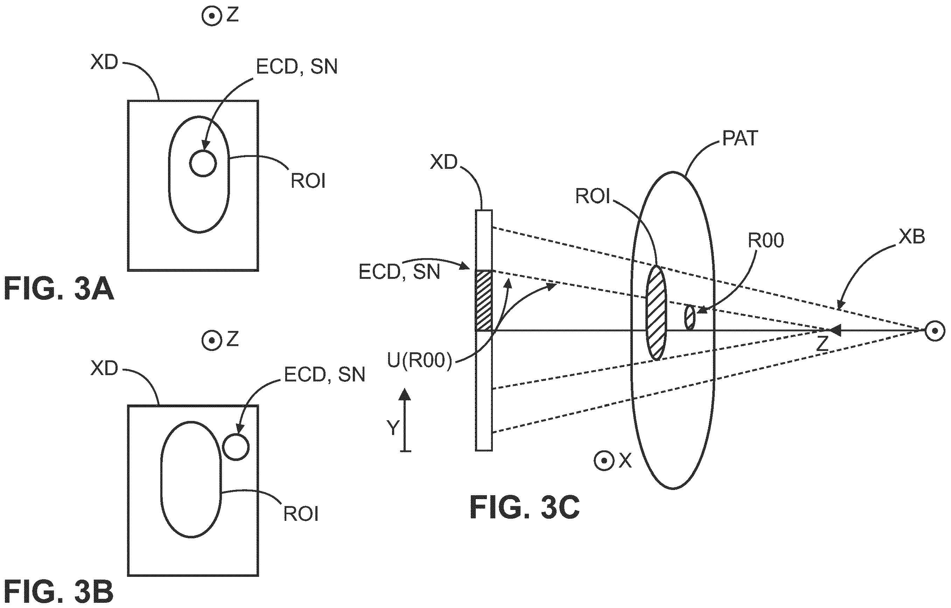

In previous imaging systems, the user was at liberty to select (activate) according to their knowledge, which subset of AEC sensor $SN_j$ are to be used during the imaging. Such selection may be done by a suitable user interface. However, such user selection was found to be error prone, as illustrated in Fig. 3.

Sometimes, especially for a novice or inexperienced user, it may not be immediately apparent which AEC sensor is to be used/activated for the given imaging task at hand. In particular, when secondary scatter radiation or patient anatomy is to be accounted for, making the correct selection may be challenging. This is illustrated by Figs. 3A and 3B. Each show projection views along the Z axis, that is, along the propagation direction of the primary beam XB. Thus, Figs. 3A, 3B show projection views of the region of interest against the X-ray detector XD's imaging surface (eg, plane). An AEC sensor may be thought to be chosen appropriately, in particular for primary X-radiation monitoring, if it overlaps in projection view with the area of the ROI, as shown in Fig 3A. Fig. 3B illustrates what can go wrong, when the position of the ECD's sensor is outside of the region of interest the projection view. Thus, position should be so that the sensor at least partly, or better fully, is within the imager's field of view ("FOV"), at least for primary radiation monitoring.

Thus, in the illustration of Fig. 3B for example, the ECD although active, may not be able to meaningfully measure the primary radiation energy deposited, as its sensor SNj is located outside the area flooded by the X-radiation. At best, it may pick up some scatter radiation. However, such a selection may not be suitable for performing the safety function of the dosage measurement system DMS correctly.

Fig. 3C also shows what can go wrong, such as when knowledge of the anatomy of the patient is not harnessed properly for imaging. Fig. 3C furnishes a side view along direction X for example, with the primary radiation direction Z arranged parallel to the drawing plane of Fig. 3C. A radiation opaque object ROO may come to lie within the X-ray beam, behind or in front of, the region of interest in projection view. Such radiation opaque object ROO or highly attenuating object may be a part of patient PAT's anatomy, such as a part of a bone. If the AEC's sensor SN happens to be within the umbra (shadow) U(ROO) that is cast by the radiation opaque object R(OO), then the reading or radiation monitored by the DMS system will underestimate the actually observed radiation, and may thus lead to false conclusions. The control module CM may thus be incorrectly instructed. That is, X-ray exposure may be allowed to continue, although it ought to have been aborted. Thus, in this case, it would have been better to choose a different AEC, one whose sensor SN is outside the umbra U(ROO) of the radiation opaque object ROO. Thus, as can be seen, the correct choice from plural distributed AECs may be a function of the medical protocol at hand, the patient's anatomy, and other factors.

Thus, as illustrated in Figs. 3, operating X-ray imager IA safely and with good purpose and results may be daunting, especially for the novice user. Because of the X-radiation's harmful health effects, it is a technique whose benefit much relies on getting it "right first time around". User must get X-ray source tube adjustments and patient positioning right, and on top of this must make the right selection of AEC's. Selecting the right AECs is not as straightforward as it may sound: selecting the wrong ones without due regard for imaging geometry and patient anatomy risks underestimating the true dosage received with detrimental health effects for patients. On the other hand, overestimating dose may cause undue interruption of imaging which reduces throughput and may cause delay and/or frustration, possibly requiring image retakes. Alas, it may not always be obvious to the novice which AECs to choose, in particular when with interferences from anatomy (Fig. 3C), or when scattering radiation, ought to be accounted for in some high scattering imaging tasks.

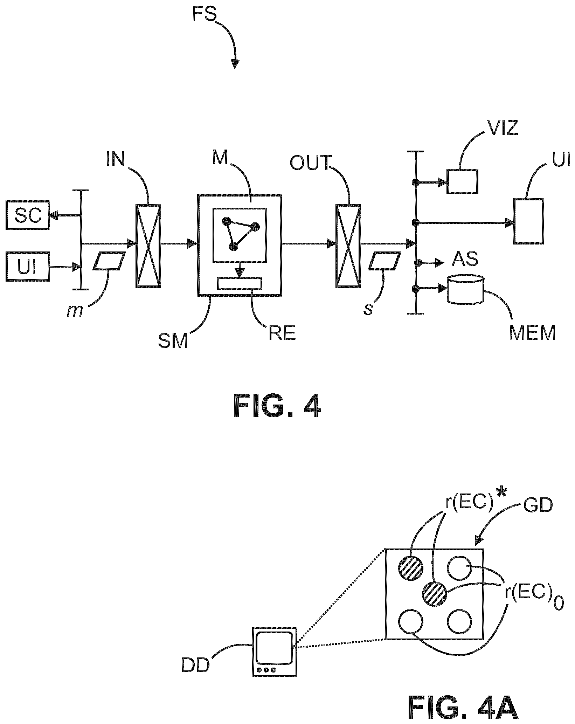

Thus, to assist the user in safe operation of the X-ray imaging apparatus, the facilitator system FS is proposed herein, whose operation will now be explained with reference to Fig. 4. The facilitator system facilitates operation of multi-AEC X-ray imagers. For example, system FS may allow automated selection out of plural AECs, of a proper, dose-safety sound, one or more AECs for the given imaging task at hand. Instead, the system FS may propose to user for the intended imaging task such a selection for approval. The system may be arranged in the operator console OC or in other computing systems such as workstation WS communicatively coupled with the X-ray imaging apparatus.

It may also be at least partly integrated into the electronics of the X-ray detector for example. It may be hard-wired or arranged in software, or both as needed. It may be arranged in a cloud or edge computing setting for distributed computing. Some of the more demanding computing tasks, in particular in machine learning embodiments, may be performed on remotely arranged more powerful computing entities, such as one or more servers etc. It is only the requesting or the sending of information on-site or the visualization of the suggested selection, etc, that is done through the computing system, operator console, or on any other computing device, such as mobile phone, tablet, laptop, etc. Alternatively, some or all functionalities are done consolidated on a single computing device. It should be noted that distributed computing may also be used by the dose monitoring system DMS. For example, the sensor(s) SN may be arranged remotely from logic CL and/or control module CM, and likewise the logic CL may be arranged remotely from the control module CM. Alternatively, logic CL and control module CM may be arranged as single computing unit, such as microcontroller, integrated in the imaging apparatus.

At one or more input ports IN of the facilitator system SC, input data $m$ is received and is then processed by an automated selector module SM to produce, at output OUT interface, selection data $s$. The selection data $s$ is indicative of the one or more automated exposure control devices ECDs that are to be used for a given imaging session, either for hard or soft activation, as mentioned above. Thus, the selection data $s$ is representative of the activation pattern that corresponds to the input data. The input data $m$ in general represents the intended imaging task for a particular patient and imaging apparatus IA having the total number of distributed ACEs. The input data may be provided by user via a user interface, such as in text form, spoken instructions, etc. In addition, or alternatively, the input data $m$ may include imagery acquired by the scout camera SC of patient PAT, and parts of the imager IA, in particular of the examination region, where at least part of the patient, in particular the ROI, resides. In addition, input data m may include any one or more of i) information regarding the patient, ii) protocol setting such as the intended anatomy, iii) the system IA configuration, etc. The selected exposure control devices AECs as per the selection data s are in general a sub-set of the total number of AEC devices that the imaging arrangement MIA or imaging apparatus IA has. The number of available AECs to choose from will depend on the make and type of the imaging arrangement MIA at hand. Thus, the selector module may be configured for a specific imaging apparatus of a specific model and make.

There may be plural such selector modules, one for each such model and make, and the user may, prior to the selector model computing such a selection of data, specify the applicable make and model of imager IA to be used for the imaging task ahead. The selection data may be visualized or may be stored in memory as needed. In particular, the selection data may be used in a user interface UI, such as a graphical UI, GUI. Such user interface UI is shown in exemplary fashion to the lower right of Fig. 4, as inset Fig. 4A.

The user interface UI may be rendered as graphical interface GUI on a display device DD, such as on a display device DD of the operator console or of workstation WS, or any other. The graphics display may represent the distribution of the selected AECs. In particular, but not necessarily, the graphics display device may include graphical indicators R(EC) that represent the individual exposure control devices AECs in whichever manner. For example, the R(EC) graphical indicators re(EC) may be rendered in a graphics display on display device DD as graphical objects, such as circles, squares or others discretely arranged shapes as schematically shown in Fig. 4A, each corresponding to a different one of the exposure devices. The selection data $s$ may be used to modulate this graphics display. For example, in the embodiment shown in Fig. 4A, there are two types of graphical indicators, one for the non-selected exposure device R(EC0) which are set off visually or otherwise against other type of graphical indicators R(EC)* that do correspond to the selected ones of the exposure devices as per the computed selection data s. For example, circles may be shown for the graphical indicators in different colors according to selection status. For example, red circles may represent selected devices AEC, whilst plain circles with no such filling or filled in a different color may represent the remaining, non-selected AECs. Any other visual modulation is also envisaged, as are non-visual clues such as spoken language instructions that indicate which ones of the automated control devices are to be chosen for the imaging session, etc, or video footage or animation, such as CGI, that illustrate the AECs to be chosen for the imaging task ahead.

The selector module SM preferably operates automatically and does not need any other input, other than the input data *m*. The input data *m* is descriptive of the imaging task ahead. For example, the input data may be textual data such as a textual prompts, which describe the imaging task to be performed. For example, user may provide as input *m* an instance of "We *would like to image for ankle fracture of patient [PPP] [with an imager of type TTT]*", where "*TTT*" stands for a particular make and model of imager IA. However, the square-bracketed portion TTT of input data may be implicit, any may not need to be provided by user. Placeholder PPP may stand for patient's bio-characteristics, such as BMI, height, weight, sex, etc. The input data *m* may be input through a user interface UI, such as textual, visual or other data. The UI may include a transducer capable of capturing user's instructions or prompts, such as via a microphone arrangement, or other.

In a preferred embodiment, the earlier mentioned scout camera is used that produces non-ionizing imagery, and it is this scout imagery which is provided as input *m* data, either on its own, or in conjunction with a textual, verbal description, such as prompts for the intended imaging task, as mentioned above. In general, the input scout imagery acquired by the one or more scout cameras SC are such that they represent the field of view of the imager. Thus, the scout camera input imagery represents at least a part of the patient such as the region of interest and at least some of the imaging geometry configuring components or their locations, such as the detector, its housing and/or the X-ray source. Line of sight may not necessarily be needed in all aspects. For example, it may be sufficient to ensure that the scout imagery merely shows the machine part that is known to include the respective component, such as the detector or other. For example, if detector is included in patient support PS, it may be sufficient for scout imagery to merely show the relevant part of the patient support PS. The scout camera SC may be integrated into the housing of the X-ray source, so that its FOV corresponds in good approximation to the X-ray FOV, although this is optional. The camera SC may be arranged, so long as the examination region is shown from whichever spatial perspective. The examination region is the portion of 3D (three-dimensional) space between imager's detector XD and the X-ray source XS, in which at least the ROI is to reside, or so resides, prior or during imaging. Such input scout imagery may represent in addition to parts of the examination region, at least a part of patient's external anatomy that relates to the region of interest, without additional imaging task description. Using such input data, the selector model SM infers the correct exposure control devices to be selected as represented by the selector data s.

In some embodiments, the facilitator system may implement an alert system which informs the user if patient anatomy is not positioned appropriately with respect to any of the available AEC sensors. Thus, in this case the selection data may indicate that no selection or activation pattern appears suitable for the given imaging task. The alert system AS may comprise a suitable transducer chain, such as visualizer VIZ generating an alert graphics display for displaying on display device DD. Alternatively, or in addition, an alarm may be sounded out via an acoustic transducer (speaker system), or a flash lamp is operated, etc.

As mentioned earlier, the selection of one or more (or all exposure devices ECD1-5, SN1-5) from a pool of plural such devices, may be formalized as an, in general, unknown latent function L of possibly many factors $\varphi$, including any one or more of the actual imaging task at hand, physiological/bio-characteristics data of the patient, the imaging apparatus type and make to be used (and hence its AECs), etc. This function *L* maps from factor space $\Phi$ into the space of selection data, L: $\Phi \in \Phi \rightarrow \Sigma \ni s$. The selection space $\Phi$ may pertain to the various AEC sensors, and in an embodiment such AEC sensor may include single one or more or groups of X-ray detector pixels. Either space $\Phi$, $\Sigma$ may be represented as a vector space or may be embedded in such. For example, it is in particular the selection data s that may be represented as a vector or matrix of suitable length or dimension. For example, data s may be a vector of a length that corresponds to the overall number *N* of exposure control devices AECs to be found in the particular imaging apparatus at hand and intended for use:

$$s = (b_1, b_2, \dots b_N), \ b_j \in \{0,1\} \tag{1}$$

Thus, selection data *s* may be coded as a one-hot or multi-hot binary vectors, made up of *0*'s and *1*'s, with each entry representing one of the AEC sensors. For example, an entry "*1*" may indicate activation of respective exposure control device AEC, whilst "*0*" represents non-activation of the respective exposure control devices. Any other coding may be used instead. In the soft activation embodiment, the b*j* are weights (numbers) in a range, such as in the open unit interval (0,1), possibly normalized, such that they sum to a constant, such as 1, eg $\Sigma_j b_j = 1$.

The selector module SM is based on a machine learning model, trained on training data to model the said function *L*. This is because certain types of machine learning models can be cast broadly without too many restrictive model assumptions, such as may be required in analytical modeling approaches. Off-the-shelf model architectures, such as of the neural network ("NN")-type with a suitable number of layers may be used. Some other hyper-parameters may be set which can be found experimentally such as the size of the convolutional filters for example, or others.

Such ML models M can then be trained on a suitable corpus of training data that can be procured by a training data procurer system DPS, either by artificially generating such data, or by sourcing from historical data. Once so procured in sufficient number and variation, with a statistical distribution that should correspond to the distribution of patients that are expected to be imaged at the medical site at which the facilitator system is to be used, the model can be so trained using a machine learning algorithm.

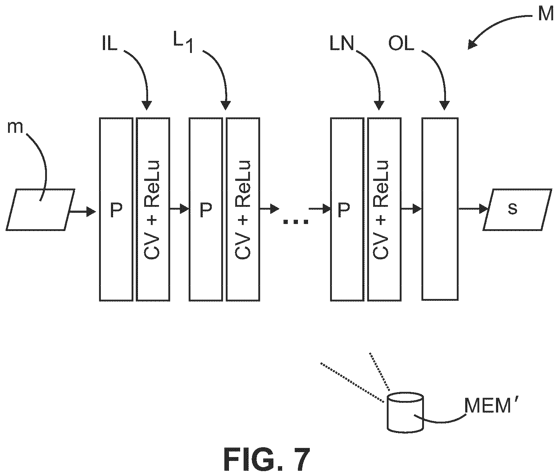

In preferred embodiments, owing to the spatial structure of the input imagery acquired by the scout camera SC, a convolutional neural network may be preferred herein, as such types models have been found to well suited to process, in particular, such spatial data. The mentioned historical training data may be procured from existing PACs or other medical databases where such historical data, in particular X-ray imagery of prior examinations, may be held. Meta data or explicit data in related data medical reports pertaining to such prior historical imaging sessions may describe which ACE sensors have been chosen. If such data is not available explicitly, historic X-ray imagery using a particular make and type of X-ray imagers may be reviewed by a human medical expert and annotated by annotation data. The annotation data awarded by reviewer upon review may define the activation pattern that ought to have been used for the given X-ray imager. In this manner, a supervised training data (x,y) can be generated by reviewer, with x indicating the training input data, and "y" the associated target. Training input data x may include historic scout imagery or other description of the imaging task eg, as per medical report as may be associated with the historic exams to be reviewed and preferably bio-characteristics of the patient that was imaged back then. The target (data) *y* may comprise the annotation data, such as the reviewer awarded activation pattern. in such embodiments, the training data procurer system DPS may include computing system with a display device for displaying of data "*x*" and suitable user input interface equipment such as pointer tool (mouse, stylus, etc) that supports an annotation tool with which reviewer can record the related target "*y*", given data "*x*". If such imagery of patient/ROI in examination region is not available in the historical data, trials can be set up to gather such scout imagery over a sufficiently long period of time, until sufficient data has accumulated. In addition or instead, such data may be gathered during clinical routine, eg to fine tune the model. This may then be reviewed by human expert. Aspects concerning machine learning are described more fully further below at Figs. 7-10.

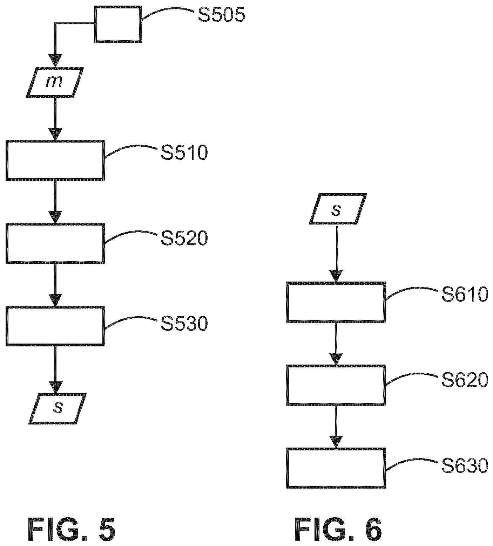

Reference is now made first to Figs. 5,6. Broadly, Fig. 5 shows a flow chart of a computer-implemented method facilitating operation of an X-ray imaging apparatus, in particular computing-assisted selection of AEC sensors SN, for use in an intended imaging task, whilst flow chart Fig. 6, downstream of the steps as per Fig. 5, illustrates use of such a selection in imaging.

In particular, and referring first to Fig. 5 in more detail, at step S505 input data *m* is generated, such as scout imagery which is acquired in a non-ionizing manner of part of the patient representative of the region of interest to be imaged for an imaging session ahead. Preferably, the scout imagery represents the ROI in the examination region of the particular imager to be used. However, other types of input data, in addition or instead of such scout imagery, may be used. Preferably, the input data *m* is such that it describes the specifics and/or objectives of the imaging task ahead. It may also include in addition or instead of such non-ionizing imagery such as optical imagery, a textual prompt or spoken instructions or any other generated by user through suitable user interfaces, such as keyboard, touch screen, sound capture device (microphonic equipment) etc. Such UIs may be supported and provided by the imager IA's operating console, or by any other computing device.

At step S510 such input data is received.

At step S520, selection data s is computed based on the input data. Preferably, a trained machine learning model is used, either end-to-end or in connection with a rule-based back-end to compute the selection data s. The machine learning model is preferably configured for multi-class classification. Suitable models may include neural networks, in particular of the convolutional type, decision trees, support vector machines, regression techniques, and others.

The selection data s to compute may be vector or of any other format, capable of representing the AEC sensors activation pattern. Such selection data *s* may be coded as a one-hot or many-hot vector or matrix, or in any other format which is capable of representing which sensors, from a known plurality of ACD sensors, are to be used for the imaging task ahead.

Such selection data *s* is then made available/output at step S530. Such selection data may then be stored, processed, displayed or otherwise used.

At step S610, and referring now to flow chart of Fig. 6 in more detail, such selection data *s* is received, previously obtained in whichever way.

At step S620 such data is presented to the user, preferably in form of a graphical user interface, or in any other way. Thus, the selection data may be suitably visualized, such as in Fig. 4A above or in any other way. The user may then issue an affirmative signal through user interface to confirm that the proposed selection is to be adopted. Alternatively, user may issue a rejection signal, with signal referring back to step S520 where new selection data is computed and presented again at step S620. The user may furnish modified or clarified input data at step S510 to so elicit computing of such new selection data. Such looping may possibly repeat, until a confirmatory signal is furnished by the user.

At step S630 the selected AEC sensors are then activated for use, and the imaging session can commence by exposure of the region of interest to acquire the imagery, with deposited X-ray dose monitored, using readings from the selected one or more AEC sensor(s).

Whilst said step S620 is mainly envisaged to support user confirmation or rejection, in principle a fully automated embodiment without the need of such user feedback, is also envisaged. In such fully automated embodiment, the selection data s is put through suitable interfacing fed as input into the imaging apparatus, such as via its operating console, to activate the selected AEC sensor. Imaging can then commence. Such fully automated embodiment may be useful in autonomous imaging set-ups. However, keeping the user in the loop such as described earlier may be the preferred option in most embodiments.

In some embodiments, no suitable selection may seem feasible, for example due to mis-positioning of ROI. In such embodiments, the selection data produced at step S630 may include an indication that no AEC selection may appear suitable. In such cases, the output data at step S630 may include an alert signal issued to inform user accordingly. Patient may then be repositioned, and the above steps - repeated, until a selection can be suggested by method.

Reference is now made to Fig. 7 which shows an architecture of a machine learning model, in particular of the convolutional neural network type, as may be envisaged herein in embodiments. Specifically, Fig. 7 shows a convolutional neural network M in a feed-forward architecture. The network M comprises a plurality of computational nodes arranged in layers in a cascaded fashion, with data flow proceeding from left to right and thus from layer to layer. Recurrent networks are not excluded herein. Convolutional networks have been found to yield good result when processing image data.

In deployment, the input data $m$ is applied to input layer IL. The input data $m$ then propagates through a sequence of hidden layers $L_1$-$L_N$ (only two are shown, but there may be merely one or more than two), to then emerge at an output layer OL as an estimate output $M(x)$. As per the embodiments described above, the output M(x) may be the sought after activation pattern s.

The model network M may be said to have a deep architecture because it has more than one hidden layers. In a feed-forward network, the "depth" is the number of hidden layers between input layer IL and output layer OL, whilst in recurrent networks the depth is the number of hidden layers, times the number of passes.

The layers of the network, and indeed the input and intermediate data (feature maps) passed between layers, can be represented as two or higher dimensional matrices ("tensors") for computational and memory allocation efficiency. The dimension of the input data, such as text or scout image, is reformed into textual output, or into output vector s that codes for the activation pattern.

Preferably, the hidden layers include a sequence of convolutional layers, represented herein as layers $L_1$ - $L_N$. The number of convolutional layers is at least one, such as 2-5, or any other number. The number may run into double-digit figures or even into the several hundreds, or higher still.

In embodiments, downstream of the sequence of convolutional layers there may be one or more fully connected layers FC, in particular if a multi-class classification result is sought as indeed envisaged herein.

Preferably, some or all of the layers are convolutional layers, that is, include one or more convolutional filters CV which process an input feature map from an earlier layer into intermediate output, sometimes referred to as logits. An optional bias term may be applied by addition for example. An activation layer processes in a non-linear manner the logits into a next generation feature map which is then output and passed as input to the next layer, and so forth. The activation layer may be implemented as a rectified linear unit RELU as shown, or as a *soft-max*-function, a sigmoid-function, *tanh*-function or any other suitable non-linear function. Optionally, there may be other functional layers such as pooling layers P or drop-out layers (not shown) to foster more robust learning. The pooling layers P reduce dimension of output whilst drop-out layer sever connections between node from different layers.

Each hidden $L_m$ layer and the input layer IL implements one or more convolutional operators CV. Each layer $L_m$ may implement the same number of convolution operators CV or the number may differ for some or all layers.

A convolutional operator CV implements a convolutional operation to be performed on its respective input. The convolutional operator may be conceptualized as a convolutional kernel. It may be implemented as a matrix including entries that form filter elements (the said weights) that form at least a part of the model parameters $\theta$. It is in particular these weights that are adjusted in the learning phase. The first layer IL processes, by way of its one or more convolutional operators, the input data such as the scout image or text or spoken language prompt. As briefly mentioned, feature maps are the outputs of convolutional layers, one feature map for each convolutional operator in a layer. The feature map of an earlier layer is then input into the next layer to produce feature maps of a higher generation, and so forth until the last layer OL represents the desired output s. In classifications, last layer OL, an output feature map of one or more previous fully connected layers FC, represents the classification result s.

It is in particular in such classification setups that the model M may include, in addition to the one or more convolutional layers IL, $L_1$-$L_N$, one or more fully connected layers FC as mentioned above. In case of a classification result, the output layer OL may be configured as a *softmax*-function layer or as similar computational nodes where feature maps from previous layer(s) are combined into normalized counts to represent the classification weights per class.

It will be understood that the above described model M in Fig. 7 is merely according to one embodiment and is not limiting to the present disclosure. What is more, models M envisaged herein are not necessarily of the neural network type at all. Other, classical statistical regression methods based on sampling from training data are also envisaged herein in alternative embodiments. Still other techniques may include Bayesian networks, or random fields, such as Markov type random field and others.

Instead of CNNs for classification or regression, also methods for image segmentation such as at least partly fully convolutional neural networks are considered. Specifically, if the detector pixels may be used as AEC sensors, the selection of the detector pixel may be conceptualized as regions of the detector pixel surface, and the selection data may then be considered as a result of a segmentation problem.

For textual input data *m,* such as natural language prompts or other, the ML model M may be of the recurrent NN type or other, such as of the transformer type. Other such generative type ML models may be used. Such generative type models, eg, text-to-text ("T2T"), such as the transformer model may comprise a tokenizer that tokenizes natural language ("NL") input imaging request into a series of tokens. The model may support an attention-mechanism that includes learnable parameters, such as attention weights. Using such attention weights, a given token may be weighted differently, depending on which position it appears in the input or intermediate input. Transformer model may have an overall NN type architecture. Transformer model may use a encoder parts and a subsequent decoder part, one or both interlaced with modules that represent the said attention mechanism. If such NL based processing is used, the output selection data s may equally be text passages describing in M which sensors are to be activated. Transformer type models were described by Ashish Vaswani et al in their paper "Attention Is All You Need", reference code arXiv: 1706.03762 [cs.CL], and available online at https://arxiv.org/abs/1706.03762.

In other embodiments, other generative type ML model may be used, such as a text-to-visual-("T2V")model. Such T2V model may be trained to receive eg, an imaging request text prompt *m,* and model provides as selection data $s=M(m)$ a visualization of the AECs to use, such as in a generated video clip. A generative multi-modal model similar to a model reported by *D Driess et al* may be used. See their paper "PaLM E: An Embodied Multimodal Language Model", reference code arXiv:2303.03378, available online at https://arxiv.org/pdf/2303.03378.

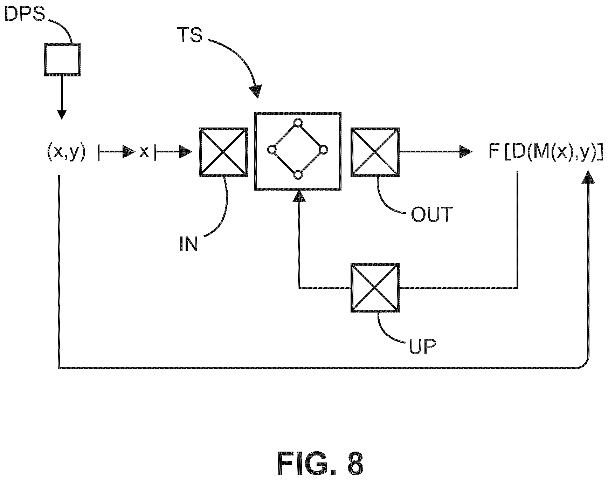

Reference is now made to Fig. 8 which shows a flow chart of a training system TS as may be used herein for training a machine learning model such as the one described above in Fig. 7.

As mentioned earlier, the set of training data $\{(x.y)\}$ may include combinations of "scout image/activations" pairs. This data can be either obtained from trained experts as mentioned earlier, or gathered in the field. Since such data is generated at a large scale during conventional clinical workflow, this approach would provide promising options for fast realization, online-learning, site specific adaptation etc. For example, if historical scout imagery as training input data is not available for a given imager, the scout camera may be readily installed in the exam room where the imager is setup, or may mounted on the imager such that FOV covers the examination region as described above. Scout imagery is then collected over time for different images sessions for different patients, to so built up a stock of such scout imagery for training input data. The clinical expert, who are familiar with the ACE sensor distribution for the given imager may then readily review such historical scout imagery field data, and awards the corresponding activation patter data as target *y,* such as in the form of vector *s* as mentioned above at (1). The reviewer may use user interface of the training data procurer DPS to populate a listing of sensors for that imager with 1's and 0', depending on whether the respective sensor should or should not be activated for the given imaging scenario as evidenced in the historical scout imagery *x*. Such an application scenario is also well suited for distributed learning scenarios (e.g. Federated Learning). Here only model updates would be exchanged between clinical sites and manufacturer.

Several extensions of this training concept can be considered herein. For example, additional context information/data c about the patient and exam could be input for the model M to learn alongside the scout imagery *x=(e, c)*.

Given historical X-ray images acquired in the previous imaging session, the training process can be further refined. The training could be restricted to data which resulted in a high image quality, as may be judged by the said historical X-ray images. Reviewer may use their clinical knowledge, and the data about patient and imager model and make (including its AEC sensor distribution) to suggest selected AEC sensors for activation that would result in meaningful measurements, and which would have been well suited for the acquisition. This expert selection may be informed by anatomical information from the X-ray Image, so so factor in for example the present of interfering objects ROO (as mentioned above at Fig. 3C) .

In a more advanced embodiment, a scene model could be derived based on the scout imagery. For some imager with a fixed (non-portable) X-ray detector for example, the exact position of the AEC chambers can be derived from the system configuration as shown in the scout imagery. At the same time, object detection and image segmentation techniques allow for the exact localization of the patient. Thus, in this case the machine learning model may be configured as a segmenter that segment for AEC position and ROI position. Thus, the output of ML model is a segmented scene, the scene being the examination region with at least parts of the patient's anatomy in it. In some cases, also the source XS and the detector XD (or the component in which it is integrated) is localized and segmented. In some embodiments, where scout camera is mounted on source XS, as indeed envisaged, the scene may not include the source as such. Thus, model M may serve as front-end in a processing chain, which feeds its scene segmentation into a rule-based system RE back-end, such as a rule or inference engine. The rules-based engine RE or similar applies clinical rules and geometric calculations as obtained from the segmented scene, to find the correct activation pattern. For example, only those AEC sensors ought to be activated that have a projective overlap with the anatomy of interest ROI (see Fig. 3A).

For a portable detector setup, we could use such an approach to localize the detector in the scout imagery scene, and to derive the location of the AEC(s) from it. In case there is no line-of-sight in respect of detector XD (e.g. detector is "hidden" behind patient is placed under tabletop of support PS, other measurements may be used in addition, such as localizer signals emittable by localizer tags, such as RFID tags used in RFID tracking, and place on or on detector XD. Such localizer input data may be used as additional input in input data x.

The above-mentioned alert functionality for inadequate patient positioning or when otherwise no suitable activation pattern can be found could be realized by introducing a further place holder class that is indicative for such inadequate positioning. In more detail, the format of the output selection data $s$ is as before at (1), but now has length $N+1$ to so code for an "enriched" vector representation $s'$, with an alert entry $a$:-

$$s' = (b_1, b_2, \ldots . b_N, a), bj \in \{0,1\} \qquad (2)$$

This, if model is configured as a multi-class classifier (as in general the AEC sensor activation will allow for selection of more than one sensor), in case of bad patient positioning, it is entry "a" that will attract most counts or weights. Thus, if placeholder entry "$a$" exceeds any other entries $bj$, or the sum of other entries $\Sigma_j b_j$, this may be taken to indicate an alert situation. A logic in alert system mas thus monitor for such an "lopsided" vector output, and, if found, issues the mentioned alert.

Turning now to the training system TS in more detail, in one or more training phases, an architecture of machine learning model M, such as the shown CNN network in Fig. 7 above is pre-populated with initial set of weights. The weights $\theta$ of the model NN represent a parameterization $M^\theta$, and it is the object of the training system TS to optimize and hence adapt the parameters $\theta$ based on the training data $\{(x,y)\}= \{(x_k, y_k)\}$ pairs. In other words, the learning can be formulized mathematically as an optimization scheme where a cost function $F$ is minimized although the dual formulation of maximizing a utility function may be used instead. If the model M includes the earlier mentioned attention mechanisms, the said trainable weights $\theta$ includes such attention weights.

Assuming for now the paradigm of a cost function $F$, this measures the aggregated residue(s), that is, the error incurred between data M(x) estimated by the model M and the targets as per some or all of the training data pairs $k$:

$$argmin_\theta F = \sum_k D[M^\theta(x_k), y_k] \qquad (3)$$

In eq. (3) and below, function $M()$ denotes the result of the model NN applied to input $x$. When configuring the model as a classifier to classify in vectors of activation patterns, the summation in (1) is formulated as one of cross-entropy or Kullback-Leiber divergence or similar. In case of using detector pixels as ACEs, ML models for image processing such as fully convolutional neural networks could be employed together with a dedicated metric (e.g. Soft-Dice). For the case of soft activation, other distance measure $D$ may be employed, such as pixel-wise squared Euclidean distance, mean squared sum, etc.

In training, the training input data $x_k$ of a training pair is propagated through the initialized network M. Specifically, the training input $x_k$ for a $k$-th pair is received at an input IL, passed through the model and is then output at output OL as output training data $M^\theta(x)$. A suitable measure $D[\cdot, \cdot]$ is used such as cross-entropy, or other, to measure the difference, also referred to herein as residue, between the actual training output $M^\theta(x_k)$ produced by the model M, and the desired target $y_k$.

The output training data $M(x_k)$ is an estimate for target $y_k$ associated with the applied input training image data $x_k$. In general, there is an error between this output $M(x_k)$ and the associated target $y_k$ of the presently considered $k$-th pair. An optimization scheme such as backward/forward propagation or other gradient based methods may then be used to adapt the parameters $\theta$ of the model M so as to decrease the residue for the considered pair $(x_k, y_k)$ or a subset (batches) of training pairs from the full training data set.

After one or more iterations in a first, inner, loop in which the parameters $\theta$ of the model are updated by updater UP for the current pair or batch (set) of pairs $(x_k, y_k)$, the training system TS enters a second, an outer, loop where a next training data pair $x^{k+1}$, $y^{k+1}$ is processed accordingly. The structure of updater UP depends on the optimization scheme used. For example, the inner loop as administered by updater UP may be implemented by one or more forward and backward passes in a forward/backpropagation algorithm. While adapting the parameters, the aggregated, for example summed, residues of all the training pairs are considered up to the current pair, to improve the objective function. The aggregated residue can be formed by configuring the objective function $F$ suitable, such as based on cross entropy or other. Other algebraic combinations instead of sums of squares are also envisaged.

Optionally, one or more batch normalization operators ("BN", not shown) may be used. The batch normalization operators may be integrated into the model M, for example coupled to one or more of the convolutional operator CV in a layer. BN operators allow mitigating vanishing gradient effects, the gradual reduction of gradient magnitude in the repeated forward and backward passes experienced during gradient-based learning algorithms in the learning phase of the model M The batch normalization operators BN may be used in training, but may also be used in deployment.

If a transformer setup is used, the cost function may include cross-entropy or variants thereof.

On transformer setup, no supervised training may be needed. Self-supervised training may be used instead, by partially making data. The training system, using a transformer model, may be made to automatically and autonomously scour imaging handbook and training material or literature in the medical field, in particular medical imaging, so build up an understanding when to use which sensors AEC. Training may proceed in staged phases where the model is first trained on mode general medical data, then on medical imaging data (handbooks, textbooks, website materials, etc) and then on handbooks for the specific model and make of the imager intended for use, which furnishes particulars on the specific AEC and their sensor distribution and use.

The training system as shown in Fig. 7 can be considered for all learning schemes, in particular supervised schemes. Unsupervised learning schemes may also be envisaged herein in alternative embodiments. GPUs may be used to implement the training system TS.

The fully trained machine learning module M may be stored in one or more memories MEM' or databases, and can be made available as pre-trained machine learning models which can be refined in federated or transfer learning setups. The trained model may be made available in a cloud service. Access can either be offered free of charge or their use can be granted via license-pay or pay-peruse scheme.

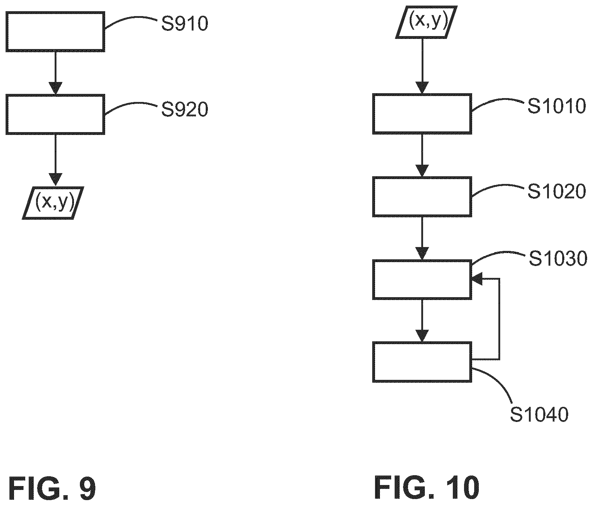

Fig. 9 shows a flow chart of a method of procuring training data whilst Fig. 10 shows a method of training a machine learning model, based on the training data so provided.

Referring now first to Fig. 9, at step S910 a request for training data is received. At step S920 training data is provided.

Step S920 may include synthetically generating suitable training data from scratch, or compiling such training data based on prior historical imaging data. In embodiments, the training data providing step S920 may include assisting a human expert (reviewer) in accessing existing training data stock, and annotating with target y the so accessed and reviewed historical data $x$ to form training data $(x,y)$ that may be used in supervised training setups. The accessing of training data may include formulating a search query in a medical database to locate such historical imaging data, in suitable number and variation. If detector pixels are to be used as AECs, the annotation may include designating detector pixel regions, eg by computer mouse or other, with the detector surface made up by such pixels suitable visualized on display device for convenience of expert, for faster, high quality annotation, and hence for better training performance of model overall.

Once such training data is procured, training method can then commence as per the flow chart of Fig. 10 to which reference is now made.

In supervised learning, the training data includes suitable pairs of data items, each pair including training input data and associated therewith a target training output data. Specifically, the pairs comprise. The scout imagery or the projection data can be paired up with expert awarded activation patterns, taking into a account imager step (make and/or model) and patient anatomy/bio-characteristics, positing in exam region, etc.

With continued reference to Fig. 10, at step S1010 training data is received in the form of pairs $(x_k, y_k)$, such as in batch (subset of all available training data pairs). Each pair includes the training input $x_k$ and the associated target $y_k$. $x_k$, as mentioned in Fig. 8 above.

At step S1020, the training input $x_k$ is applied to an initialized machine learning model to produce a training output.

A deviation, or residue, of the training output $M(x_k)$ from the associated target $y_k$ is quantified by a cost function $F$. One or more parameters of the model are adapted at step S1030 in one or more iterations in an inner loop to improve the cost function. For instance, the model parameters are adapted to decrease residues as measured by the cost function. The parameters include in particular weights of the convolutional operators, in case a convolutional NN model M is used, however such parameters may refer to different entities, given the particulars of the model.

Whilst the steps above may relate to a supervised training system, the principles described herein are not so confined. Unsupervised, semi-supervised or reinforced training is also envisaged herein, where the pairings are found automatically by the training system in analyzing large amount of training data.

The components of system FS may be implemented as one or more software modules, run on one or more general-purpose processing units PU such as a workstation WS associated with the imager IA, or on a server computer associated with a group of imagers.

Alternatively, some or all components of system FS may be arranged in hardware such as a suitably programmed microcontroller or microprocessor, such an FPGA (field-programmable-gate-array) or as a hardwired IC chip, an application specific integrated circuitry (ASIC), integrated into the imaging system MIA, IA. In a further embodiment still, the system FS may be implemented in both, partly in software and partly in hardware.

The different components of the system FS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

**Claims**

1. A system (FS) of facilitating operation of a medical X-ray imaging apparatus having plural exposure control devices (SN, ECD1-5), comprising:

   an input interface (IN) through which is receivable input data (m) pertaining to an acquisition operation to be performed in relation to a region of interest (ROI);
   a selector module (SM) capable to provide a selection (s), based on the input data, of one or more of the plural exposure control devices (SN, ECD1-5) for activation during the acquisition operation,
   wherein the selector module (SM) is based on a trained machine learning model (M).

2. The system of any one of the preceding claims, wherein the selector module (SM) is based on a rule-based decision logic.

3. The system of any one of the preceding claims, wherein the system is capable of providing output data through an output interface (OUT), the said output data indicative to the said selection.

4. The system of any one of the preceding claims, wherein the output data includes a graphics display (GD) capable of visualizing, on a display device (DD), the said output data.

5. The system of any one of the preceding claims, including a user interface (UI) capable of allowing user to confirm the said selection.

6. The system of any one of the preceding claims, wherein the input data includes non-ionizing radiation-based imagery acquirable by a scout camera (SC) of at least a part of the region of interest.

7. The system of any one of the preceding claims, wherein the region of interest (ROI) includes at least a part of an anatomy of a patient (PAT) to be imaged.

8. An imaging arrangement (MIA), comprising the system (FS) of any one of the preceding claims, and further comprising: i) the imaging apparatus (IA), ii) the user interface (UI); iii) the display device (DD), iv) the scout camera (SC), v) at least one of the plural exposure control devices (ECD1-3).

9. A method for facilitating operation of a medical X-ray imaging apparatus having plural exposure control devices, comprising:

   receiving (S510) input data pertaining to an acquisition operation to be performed in relation to a region of interest; and
   selecting (S520) a selection, based on the input data, of one or more of the plural exposure control devices for activation during the acquisition operation,
   wherein the selecting (S520) is based on a trained machine learning model (M).

10. A training method for training, based on training data, the machine learning model (M) as per claim 1.

11. A method of providing the training data for use in the method of claim 10.

12. A computer program element, which, when being executed by at least one processing unit, is adapted to cause the processing unit to perform the method as per claim 9, 10 or 11.

13. At least one computer readable medium having stored thereon the program element of claim 12, or having stored thereon the trained machine learning model as per claim 2.

**FIG. 1**

**FIG. 1A**

**FIG. 2**

FIG. 3C

FIG. 3A

FIG. 3B

**FIG. 4**

**FIG. 4A**

**FIG. 5**

**FIG. 6**

**FIG. 7**

**FIG. 8**

**FIG. 9**

**FIG. 10**

Europäisches
Patentamt
European
Patent Office
Office européen
des brevets

# EUROPEAN SEARCH REPORT

**Application Number**

EP 24 18 9562

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/365227 A1 (KAWAI MASAYA [JP] ET AL) 17 November 2022 (2022-11-17) * paragraphs [0021], [0036], [0053], [0054], [0056]; figures 1,3 * | 1-13 | INV. A61B6/46 A61B6/00 |
| A | CN 104 545 957 A (SIEMENS SHANGHAI MED EQUIP LTD) 29 April 2015 (2015-04-29) * the whole document * | 1-13 | |
| A | US 2024/138801 A1 (NISHIJIMA KATSUHIRO [JP] ET AL) 2 May 2024 (2024-05-02) * the whole document * | 1-13 | |
| A | US 2024/161274 A1 (XUE PING [US] ET AL) 16 May 2024 (2024-05-16) * the whole document * | 1-13 | |

**TECHNICAL FIELDS SEARCHED (IPC)**

A61B

The present search report has been drawn up for all claims

| Place of search | Date of completion of the search | Examiner |
|---|---|---|
| Munich | 12 December 2024 | Marzal-Abarca, X |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 18 9562

This annex lists the patent family members relating to the patent documents cited in the above-mentioned European search report.
The members are as contained in the European Patent Office EDP file on
The European Patent Office is in no way liable for these particulars which are merely given for the purpose of information.

12-12-2024

| Patent document cited in search report | | Publication date | Patent family member(s) | | Publication date |
|---|---|---|---|---|---|
| US 2022365227 | A1 | 17-11-2022 | JP | 2022174663 A | 24-11-2022 |
| | | | US | 2022365227 A1 | 17-11-2022 |
| | | | US | 2024353580 A1 | 24-10-2024 |
| CN 104545957 | A | 29-04-2015 | NONE | | |
| US 2024138801 | A1 | 02-05-2024 | EP | 4360562 A1 | 01-05-2024 |
| | | | JP | 2024064987 A | 14-05-2024 |
| | | | US | 2024138801 A1 | 02-05-2024 |
| US 2024161274 | A1 | 16-05-2024 | CN | 118021339 A | 14-05-2024 |
| | | | US | 2024161274 A1 | 16-05-2024 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. M. MITCHELL**. Machine Learning. McGraw-Hill, 1997, 2
- **ASHISH VASWANI et al.** Attention Is All You Need. *arXiv: 1706.03762*, https://arxiv.org/abs/1706.03762
- **PALM E**. An Embodied Multimodal Language Model. *arXiv:2303.03378*, https://arxiv.org/pdf/2303.03378